# EUROPÄISCHE PATENTSCHRIFT

(11) **EP 0 664 989 B1**
(45) Veröffentlichungstag und Bekanntmachung des Hinweises auf die Patenterteilung: **03.07.2002**
(21) Anmeldenummer: 94117390.8
(22) Anmeldetag: 04.11.1994
(51) Int. Cl.: A61B 5/00

(54) **Anordnung zur kontinuierlichen Überwachung der Konzentration eines Metaboliten**
Instrument for continuously monitoring the concentration of a metabolit
Dispositif de surveillance continue de la concentration d'un métabolite

(30) Priorität: 19.01.1994 DE 4401400
(43) Veröffentlichungstag der Anmeldung: 02.08.1995
(73) Patentinhaber: Institut für Diabetestechnologie, Gemeinnützige Forschungs- und Entwicklungsgesellschaft mbH an der Universität Ulm, 89081 Ulm (DE)
(72) Erfinder: Pfeiffer, Ernst, Prof. Dr., D-89075 Ulm (DE); Sternberg, Fabio, Dr., D-89231 Neu-Ulm (DE)
(74) Vertreter: Wolf, Eckhard, Dr.-Ing.

(56) Entgegenhaltungen:
- EP-A- 0 401 179
- EP-A- 0 409 467
- EP-A- 0 534 074
- WO-A-89/02720

## Beschreibung

Die Erfindung betrifft eine Anordnung zur kontinuierlichen Überwachung der Konzentration eines Metaboliten, wie Glukose oder Milchsäure, in Biogeweben.

Anordnungen dieser Art finden vor allem im Bereich der Humanmedizin ihre Verwendung. Neben sportmedizinischen Anwendungen wie der Milchsäureüberwachung unter anaerober Belastung oder der Bestimmung der Variation freier Fettsäuren unter verschiedenen Bedingungen steht hier vor allem die Blutzuckerüberwachung von Diabetikern im Vordergrund. Eine schnelle Information über den Blutzuckerspiegel ist unumgängliche Bedingung für eine effektive, rückgekoppelte Dosierung von Insulingaben. Unter außerklinischen Bedingungen des täglichen Lebens kommt jedoch eine vom Patienten durchgeführte Selbstüberwachung des Zuckerspiegels mittels kontinuierlich am venösen Blutstrom angekoppelter Biosensoren unter anderem wegen Gerinnungsproblemen kaum in Frage. Als gangbare Alternative hat sich die Bestimmung des mit dem Blutzuckerspiegel weitgehend linear korrelierten Zucker- bzw. Glukosegehalts der interstitiellen Gewebeflüssigkeit herausgestellt. Bei den ersten nach diesem Prinzip arbeitenden Verfahren (vgl. EP-A-275 390) wurde ein mit einem immobilisierten Enzym beschichteter Nadelsensor in das Gewebe eingebracht und der Glukosegehalt ohne Probenentnahme in situ direkt in der Interstitialflüssigkeit durch Messungen auf enzymatisch-elektrochemischer Basis bestimmt. Dieses Konzept erwies sich jedoch als nachteilig, da bei Langzeitmessungen in vivo die Glukosesensivität des Nadelsensors durch Einflüsse physiologischer Parameter mit der Zeit stetig abnahm. Um dem zu entgehen, wurde vorgeschlagen (DE-A-41 30 742), Metaboliten mittels der Mikrodialysetechnik aus der Gewebeflüssigkeit zu gewinnen, die eigentliche Messung aber nach ex vivo zu verlagern, wobei ein Probentransport von der subkutan implantierten Mikrodialysesonde zu einer extrakorporal angeordneten elektrochemischen Enzymzelle über eine Dialysatleitung erfolgt. Mit dieser Technik war es unter zusätzlicher Spülung der Enzymzelle mit Pufferflüssigkeit möglich, den Einfluß von in biologischem Material vorhandenen Enzyminhibitoren zurückzudrängen und die Validität von Langzeitmessungen zu erhöhen. Intravenöse Referenzmessungen zeigten eine hohe Korrelation und eine unter Normalbedingungen geringe zeitliche Verzögerung zwischen den Zuckerspiegeln von Blut und Gewebe. Allerdings wurde eine mit dem allmählichen Verlust an Enzymaktivität verbundene Meßsignaldrift beobachtet. Zugleich erforderten Herstellung und Austausch des enzymbeschichteten Sensors vergleichsweise hohe Materialkosten.

Um diese Nachteile zu vermeiden, sieht eine Anordnung gemäß der WO 89/02720 vor, daß eine Enzymlösung von vorgegebener Konzentration als Perfusionsflüssigkeit durch die Mikrodialysesonde hindurchgeleitet wird. Alternativ wird dort ein das Enzym enthaltender, zwischen der Sonde und der Meßelektrode angeordneter Reaktionsraum vorgeschlagen. Als Reaktionsraum ist eine perfusatdurchflossene Hohlfaser vorgesehen, durch welche das Enzym hindurchdiffundieren soll. Das Enzym wird dabei in fester Form mantelseitig an der Hohlfaser vorgehalten.

Hiervon ausgehend liegt der Erfindung die Aufgabe zugrunde, die bekannte Anordnung so zu verbessern, daß stabile und genaue Langzeitmessungen der Konzentration von Metaboliten in Biogeweben auf Online-Basis ermöglicht werden, wobei eine kompakte und kostensparende Bauart der Einzelkomponenten, einfache Handhabung und hohe Biokompatibilität gefordert wird.

Zur Lösung dieser Aufgabe werden die in den Ansprüchen 1 bzw. 14 angegebenen Merkmalskombinationen vorgeschlagen. Weitere vorteilhafte Ausgestaltungen und Weiterbildungen der Erfindung ergeben sich aus den abhängigen Ansprüchen.

Die erfindungsgemäße Lösung geht von dem Gedanken aus, daß zur Kostenverringerung und Stabilitätsverbesserung des Meßsensors die enzymatisch katalysierten Reaktionsvorgänge unter räumlicher Trennung vom Meßsensor und unter stetiger oder gelegentlicher Erneuerung des Enzyms ablaufen sollten.

Eine bevorzugte Ausgestaltung der Erfindung sieht vor, daß die Enzymlösung als kontinuierlicher Enzymlösungsstrom stromauf der Meßstelle dem Dialysatstrom zugemischt wird, und daß mit dem so gebildeten Meßdialysatstrom ein an der Meßstelle angeordneter elektrochemischer Meßsensor kontinuierlich beaufschlagt wird. Dadurch werden Meßproben mit gleichbleibender Enzymaktivität stetig an dem Meßsensor vorbeigeführt und eine mit den Stoffwechselvorgängen schritthaltende, bei zu vernachlässigender Fließdauer in "Echtzeit" ablaufende Meßführung ermöglicht. Die Konzentration des Metaboliten im Meßdialysatstrom wird dann vorteilhafterweise dadurch bestimmt, daß der Metabolit im Meßdialysatstrom unter Einwirkung des Enzyms oxidiert wird, und daß die Konzentration eines an der Oxidationsreaktion teilnehmenden oder dabei entstehenden Reaktionspartners mittels zweier polarisierter Elektroden des Meßsensors amperometrisch bestimmt wird.

Um die Elektroden des Meßsensors vor Ablagerungen zu schützen und die enzymatisch induzierten Vorgänge von den elektrochemischen Vorgängen zu trennen, werden die Elektroden des Meßsensors mittels einer für den Reaktionspartner durchlässigen, vorzugsweise als für das Enzym undurchlässige Dialysemembran ausgebildeten Schutzmembran von dem Meßdialysatstrom getrennt. Zur Senkung von Verbrauchskosten wird die Schutzmembran zweckmäßig auswechselbar in dem Sensor angeordnet.

Eine gleichbleibende Enzymkonzentration im Meßdialysat kann dadurch erreicht werden, daß der Enzymlösungsstrom in Abhängigkeit vom Durchsatz der Mikrodialysesonde zugemischt wird, wobei das Verhältnis zwischen Perfusatstrom und Enzymlösungsstrom 1:10 bis 10:1, vorzugsweise 1:1 beträgt.

Eine ausreichende Anreicherung des Perfusatstroms mit Metaboliten wird erzielt, wenn der Perfusatstrom 1 bis 15 µl/min, vorzugsweise 6 bis 7 µl/min beträgt.

Je nach Anwendungsfall kann als Enzym beispielsweise Glukoseoxidase (zur Bestimmung von Glukose) oder Laktatoxidase (zur Bestimmung von Laktat) eingesetzt werden. Vorteilhafterweise beträgt die Konzentration des Enzyms in der Enzymlösung von 100 U/ml bis 10000 U/ml.

Eine bioverträgliche Perfusionsflüssigkeit besteht beispielsweise aus physiologischer Kochsalz- oder Ringerlösung, die zur Stabilisierung des pH-Werts phosphatgepuffert sein kann. Ferner kann die Enzymlösung zur Ausschaltung eventueller bakterieller Einflüsse auf die Messung mit Kresolen in einer Konzentration von 0,01 bis 1 Gew.% versetzt werden.

Um die Linearität der Messung auch bei höheren Metabolitkonzentrationen zu gewährleisten, wird die Enzymlösung und/oder die Perfusionsflüssigkeit mit Sauerstoff angereichert, indem sie beispielsweise vor der Messung einer Sauerstoff-Atmosphäre ausgesetzt wird.

Bei einer zur Durchführung des erfindungsgemäßen Verfahrens geeigneten bevorzugten Anordnung wird zur Lösung der vorstehend angegebenen Aufgabe vorgeschlagen, daß die Dialysat- oder/und Perfusatleitung aus einem Enzymlösungsreservoir mit einer Enzymlösung beaufschlagbar ist, und daß die Meßanordnung einen zur Bestimmung der Konzentration des unter der Einwirkung des Enzyms oxidierbaren Metaboliten ausgebildeten, an der Dialysatleitung angekoppelten Meßsensor aufweist. Damit ist es möglich, die Enzymlösung nach Bedarf zuzumischen, und einen kostengünstig herstellbaren, für verschiedene Enzyme einsetzbaren Meßsensor zu verwenden.

Gemäß einer vorteilhaften Ausgestaltung der Erfindung mündet das Enzymlösungsreservoir über einen Zuführkanal stromauf des Meßsensors an einer Mischstelle zusammen mit der Dialysatleitung in eine Meßdialysatleitung. Durch den gemeinsamen Transport in der Meßdialysatleitung wird eine ausreichende Wechselwirkung zwischen dem Enzym und dem Metabolit vor dem eigentlichen Nachweis gewährleistet. Gleichzeitig wird vermieden, daß Enzymlösung durch die Mikrodialysesonde hindurchgeschleust wird, so daß bei einer Beschädigung der Dialysemembran kein artfremdes Eiweiß aus der Mikrodialysesonde in das Körpergewebe gelangen kann.

Ein konstanter Perfusatstrom kann durch eine in der Perfusatleitung angeordnete, eingangsseitig mit einem Perfusatreservoir und ausgangsseitig mit der Mikrodialysesonde kommunizierende erste Fördereinheit erzeugt werden. Um den mit dem Metaboliten angereicherten Perfusatstrom aus der Mikrodialysesonde als Dialysatstrom abzuführen und unter konstanter Zumischung von Enzymlösung als Meßdialysatstrom an der Meßsonde vorbeizuführen, kann eine zweite Fördereinheit stromab der Mischstelle vor oder nach dem Meßsensor in der Meßdialysatleitung angeordnet werden.

Ein Rückströmen von Enzymlösung in die Mikrodialysesonde wird bevorzugterweise dadurch verhindert, daß die Förderleistung der zweiten Fördereinheit größer, vorzugsweise doppelt so groß wie die Förderleistung der ersten Fördereinheit ist.

Um eine exakte Proportionalität zwischen Perfusatstrom und Meßdialysatstrom und damit konstante Meßbedingungen zu erzielen, sind die erste und zweite Fördereinheit jeweils als Dosierpumpe, vorzugsweise als Rollendosierpumpe oder Kolbenpumpe ausgebildet.

Ein besonders kompakter Aufbau wird dadurch erreicht, daß die erste und zweite Fördereinheit als in der Perfusat- und Meßdialysatleitung angeordnete, durch einen gemeinsamen Rollkolben zusammendrückbare Pumpschläuche einer einzelnen Rollendosierpumpe ausgebildet sind. Unterschiedliche Durchflußmengen lassen sich dabei durch verschiedenlumige Pumpschläuche einstellen.

Um einen quantitativen Nachweis des Metaboliten im Meßdialysatstrom zu ermöglichen, kann ein für amperometrische Messungen ausgebildeter Meßsensor verwendet werden, der eine vorzugsweise aus Platin oder Gold bestehende Meßelektrode und eine der Meßelektrode benachbarte, vorzugsweise aus Silber oder Edelstahl bestehende Vergleichselektrode aufweist.

Um die enzymatischen Vorgänge von den elektrochemischen Vorgängen an den Elektroden zu trennen und Ablagerungen auf denselben zu verhindern ist es von Vorteil, wenn der Meßsensor eine die Elektroden vom Meßdialysatstrom trennende, zweckmäßig auswechselbare semipermeable Schutzmembran aufweist.

Zur Optimierung der Meßbedingungen und zur Verringerung von Signaldrift kann eine die Temperatur in der Durchflußkammer auf einen vorzugsweise 29°C betragenden Vorgabewert einregelnde Temperaturregeleinrichtung vorgesehen werden.

Eine Rückströmung von enzymhaltigem Meßdialysat in Richtung der Mikrodialysesonde kann durch ein in der Dialysatleitung angeordnetes, selbsttätig sperrendes Rückschlagventil verhindert werden.

Der erfindungsgemäße Meßsensor wird vorzugsweise in Verbindung mit einer zweckmäßig an einem Patienten tragbaren Meßanordnung eingesetzt, die eine das Meßsignal des Meßsensors aufnehmende und daraus mit der Konzentration des Metaboliten im Gewebe korrelierte digitale Einzeldaten generierende, vorzugsweise mikroprozessorgesteuerte Auswerteelektronik aufweist.

Die auf diese Weise gewonnenen digitalen Einzeldaten können zur Erstellung eines Langzeitbildes in kontinuierlichen Zeitabständen in einem Speichermedium abgespeichert werden und zur Ablesung durch den Patienten an einem vorzugsweise als LCD-Anzeige ausgebildeten Anzeigemittel aktuell angezeigt werden.

Die erfindungsgemäße Anordnung findet vorteilhafterweise zum Ausgleich diabetischer Defekte bei zuckerkranken Patienten Verwendung, wobei in Kombination mit einem automatisch arbeitenden Insulin-Infusionsmittel eine Steuerung der Insulingabe nach Maßgabe der im Körpergewebe des Patienten bestimmten Glukosekonzentration erfolgt.

Im folgenden wird die Erfindung anhand eines in der Zeichnung schematisch dargestellten Ausführungsbeispiels näher erläutert. Es zeigen
- Fig. 1: ein Blockschaltbild einer Meßanordnung zur Überwachung von Metaboliten in Körpergewebe;
- Fig. 2: eine Mikrodialysesonde in vereinfachter geschnittener Darstellung;
- Fig. 3a und b: einen Meßsensor und eine die Durchflußkammer begrenzende Platte in schaubildlicher Explosionsdarstellung und zusammengebaut in geschnittener Darstellung.

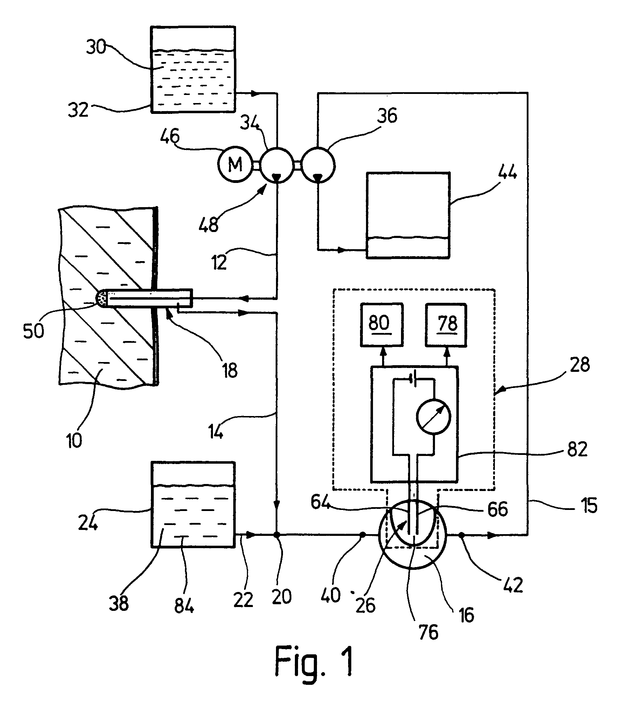

Die Anordnung zur Überwachung von Metabolitkonzentrationen im Körpergewebe gemäß Fig. 1 besteht im wesentlichen aus einer in das Subkutangewebe 10 eines Patienten implantierbaren Mikrodialysesonde 18 und einer extrakorporal angeordneten Meßanordnung 28, wobei die eingangsseitig über eine Perfusatleitung 12 mit einem Perfusatstrom beaufschlagbare Mikrodialysesonde 18 ausgangsseitig über eine Dialysatleitung 14 und eine Meßdialysatleitung 15 mit einer Durchflußkammer 16 kommuniziert, an die ein Meßsensor 26 der Meßanordnung 28 angekoppelt ist, und wobei an einer Mischstelle 20 zwischen der Dialysatleitung 14 und der Meßdialysatleitung 15 ein Zuführkanal 22 eines Enzymlösungsreservoirs 24 angeschlossen ist.

Die beispielsweise aus phosphatgepufferter physiologischer Kochsalzlösung oder Ringer-Lösung bestehende Perfusionsflüssigkeit 30 wird mittels einer in der Perfusatleitung 12 angeordneten, eingangsseitig aus einem Perfusatreservoir 32 gespeisten ersten Fördereinheit 34 der Mikrodialysesonde 18 zugeführt. Eine stromab der Mischstelle 20 und der Durchflußkammer 16 nachgeschaltet angeordnete zweite Fördereinheit 36 sorgt aufgrund ihrer im Vergleich zur ersten Fördereinheit 34 größeren Förderleistung dafür, daß der mit Metaboliten angereicherte Perfusatstrom als Dialysatstrom aus der Mikrodialysesonde 18 abgesaugt und unter zusätzlicher Zumischung von Enzymlösung 38 an der Mischstelle 20 als Meßdialysatstrom durch die Meßdialysatleitung 15 weitergeleitet wird. Dabei wird durch die höhere Förderleistung der zweiten Fördereinheit 36 gewährleistet, daß keine Enzymlösung 38 von der Mischstelle 20 in Richtung der Mikrodialysesonde 18 fließen kann. Der Meßdialysatstrom wird durch die an Anschlußstellen 40,42 in die Meßdialysatleitung 15 geschaltete Durchflußkammer 16 hindurch- und dabei am Meßsensor 26 vorbeigeleitet und in einen der zweiten Fördereinheit 34 nachgeordneten Auffangbehälter 44 zur späteren Entsorgung abgeleitet. Die erste und zweite Fördereinheit 34,36 können als voneinander getrennte Dosierpumpen, oder - wie in dem hier beschriebenen Ausführungsbeispiel - als durch einen gemeinsamen, mittels eines zweckmäßig batteriegespeisten Motors 46 angetriebenen Rollkolben zusammendrückbare Pumpschläuche einer Rollendosierpumpe 48 ausgebildet sein. Die auf die Perfusatleitung 12 und die Meßdialysatleitung 15 gemeinsam einwirkende Rollendosierpumpe 48 gewährleistet mit einfachen Mitteln die Einstellung eines vorgegebenen Verhältnisses zwischen Perfusatstrom und Meßdialysatstrom nach Maßgabe der verwendeten Pumpschlauchquerschnitte.

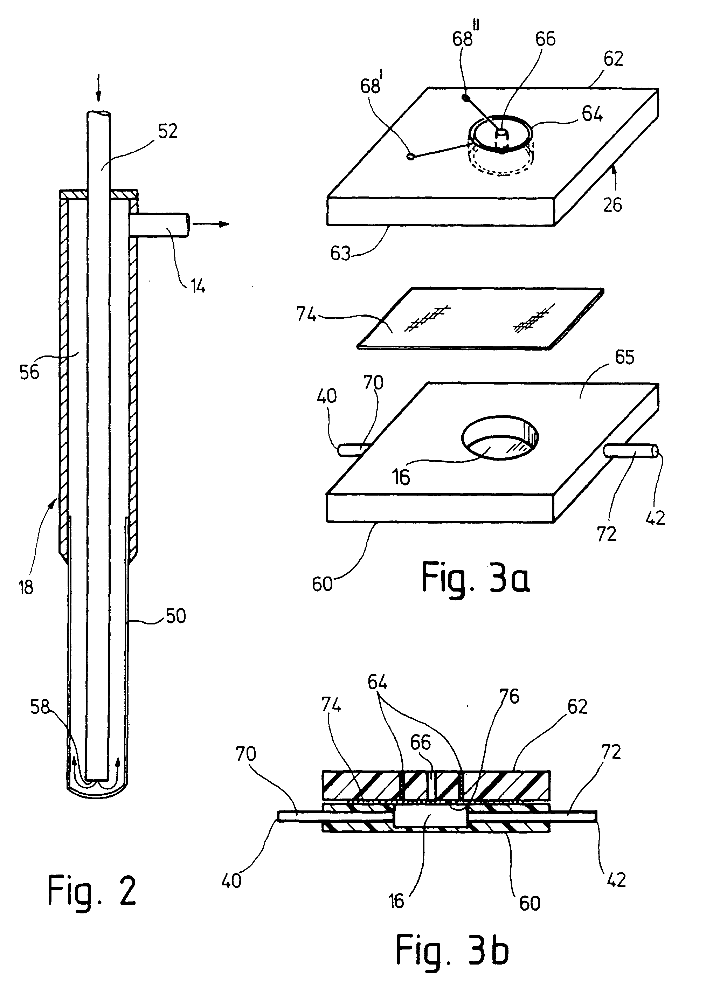

Wie aus Fig. 2 ersichtlich, besteht die Mikrodialysesonde 18 im wesentlichen aus einer an ihrem proximalen und distalen Ende verschlossenen doppellumigen Nadel mit einer Feinheit von etwa 20 Gauge, die im Bereich ihres distalen Endes mit einer Dialysemembran 50 versehen ist und die eine zentral angeordnete, mit der Perfusatleitung 12 kommunizierende innere Nadelkanüle 52 aufweist, die bis zum distalen Ende hindurchgreift. Die gleichfalls am proximalen Ende herausgeführte Dialysatleitung 14 reicht über einen Ringkanal 56 bis zum distalen Nadelende und kommuniziert dort mit der Auslaßöffnung 58 der inneren Nadelkanüle 52. Der Membranteil 50 der Dialysesonde ist bei deren Implantation vollständig in das Gewebe eingebettet, wobei die Porosität der Dialysemembran 50 so bemessen ist, daß die zu bestimmenden Stoffwechselprodukte, wie Glukose oder Milchsäure nahezu widerstandsfrei durch die Membran 50 hindurchdiffundieren können, während größere Moleküle zurückgehalten werden. Zur Glukose- und Milchsäurebestimmung wird die Porenweite bei 0,01 bis 0,03 µm gewählt. Aufgrund des zwischen der Interstitialflüssigkeit und dem an der Membran 50 entlanggepumpten Perfusatstrom hinsichtlich der Metaboliten bestehenden Konzentrationsgradienten wird das Perfusat mit Metaboliten aus dem interzellularen Gewebe befrachtet. Daraus resultiert ein Dialysat, wobei der Konzentrationsgradient durch Abpumpen des Dialysats kontinuierlich aufrechterhalten wird.

Der in Fig. 3a zusammen mit einer die Durchflußkammer 16 begrenzenden Kammerplatte 60 schaubildlich dargestellte elektrochemische Meßsensor 26 weist eine Isolatorplatte 62 auf, in die eine hohlzylindrische Vergleichselektrode 64 aus Silber oder Edelstahl sowie eine stiftförmige Meßelektrode 66 aus Platin oder Gold in zueinander koaxialer Anordnung über deren Breitseitenfläche 63 überstehend eingebettet sind. Die Isolatorplatte weist ferner zwei jeweils mit einer der voneinander isolierten Elektroden 64,66 elektrisch leitend verbundene Anschlußstellen 68', 68'' auf. Die mit der Isolatorplatte verbindbare Kammerplatte 60 ist mit einer die Durchflußkammer 16 bildenden, zentral angeordneten und zu einer Breitseitenfläche 65 hin randoffenen zylindrischen Ausnehmung versehen. Des weiteren wird die Kammerplatte 60 von zwei an gegenüberliegenden Schmalseitenflächen überstehenden, in die Durchflußkammer 16 mündenden Verbindungsrohren 70,72 durchsetzt, die zum Anschluß der Durchflußkammer 16 an die Anschlußstellen 40,42 der Meßdialysatleitung 15 dienen. Gegebenenfalls kann die Durchflußkammer 16 bzw. das durch sie hindurchströmende Meßdialysat mittels einer nicht dargestellten Temperaturregeleinrichtung auf eine vorgebbare Temperatur eingeregelt werden.

Die Isolatorplatte 62 und die Kammerplatte 60 können unter gegenseitiger Anlage ihrer Breitseitenflächen 63 bzw. 65 durch nicht dargestellte Verbindungsmittel lösbar miteinander verbunden werden. Beim Zusammenbau wird zwischen der Kammerplatte 60 und der Isolatorplatte 62 eine die Durchflußkammer 16 an ihrer den Elektroden 64,66 zugewandten Öffnung überdeckende, vorzugsweise als Dialysemembran ausgebildete Schutzmembran 74 eingelegt und eingeklemmt (Fig. 3b). Auf diese Weise läßt sich die Schutzmembran 74 bei Bedarf einfach auswechseln. In zusammengebautem Zustand befindet sich dann die aktive Meßstelle 76 des Meßsensors 26 im Bereich zwischen den durch die Schutzmembran 74 abgedeckten Stirnflächen der Elektroden. Die Vergleichselektrode 64 und die Meßelekrode 66 sind über die Anschlußstellen 68',68'' an eine in Fig. 1 symbolisch dargestellte, mit einem Anzeigemittel 78 und einem Speichermedium 80 verbundene Auswerteelektronik 82, wie sie beispielsweise in der EP-A-275 390 beschrieben ist, anschließbar und mit einer Gleichspannung beaufschlagbar.

Bei der Meßdurchführung dient das in der Durchflußkammer 16 am Meßsensor 26 vorbeigeführte Meßdialysat als Elektrolyt. Auf der Fließstrecke zwischen der Mischstelle 20 und der Meßstelle 76 bewirkt eine zugemischte Enzymlösung 38, die Glukoseoxidase als Enzym 84 enthält, daß die aus dem Gewebe gewonnene β-D-Glukose im Beisein von Sauerstoff unter Freisetzung von H₂O₂ zu D-Glukonolakton oxidiert, welches nachfolgend in wäßriger Lösung hydrolytisch zu D-Glukonsäure reagiert:

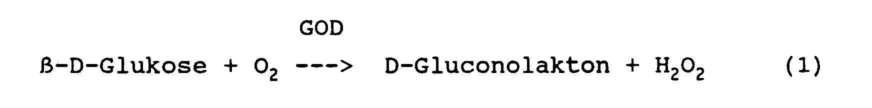

Die Bildungsrate von H₂O₂ kann als Diffusionsgrenzstrom aufgrund der folgenden Reaktion an der Platinanode amperometrisch gemessen werden:

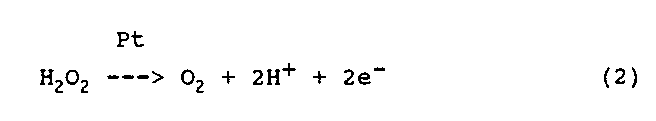

Die zu bestimmende Glukosekonzentration verhält sich proportional zu dem so gemessenen Strom. Der im Meßdialysat vorhandene Sauerstoff reicht für den Reaktionsablauf aus, zumal der bei der Glukose-Umwandlung verbrauchte Sauerstoff bei der Umwandlung von H₂O₂ an der Pt-Anode 66 zurückgewonnen und zumindest teilweise in das Meßdialysat zurückgeführt wird. Zusätzlich ist es jedoch möglich und insbesondere bei hohen Metabolitkonzentrationen zweckmäßig, die Enzymlösung 38 oder die Perfusionsflüssigkeit 30 mit Sauerstoff anzureichern.

Zum Nachweis von Milchsäure (Laktat) kann bei grundlegend gleichem Meßaufbau mit einer Enzymlösung 38, die Laktatoxidase als Enzym 84 enthält, in dem Meßdialysat vorhandenes Laktat im Beisein von Sauerstoff in Pyruvat (Brenztraubensäure) unter Freisetzung von H₂O₂ oxidiert werden:

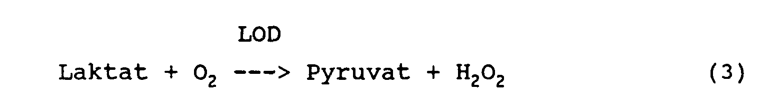

Auch hier kann die Bildungsrate von H₂O₂ mittels des elektrochemischen Sensors als Diffusionsgrenzstrom im Sinne der vorstehenden Reaktionsgleichung (2) an der Platinanode 66 gemessen werden.

Sowohl beim Glukosesensor als auch beim Laktatsensor wird der Diffusionsgrenzstrom mit einer positiven Polarisationsspannung gemessen.

Grundsätzlich ist es möglich, den in Fig. 1 gezeigten Sensor auch mit entgegengesetzter Polarisationsspannung, also mit dem Platinstift 66 als Kathode und der zylindrischen Silberelektrode 64 als Anode zu betreiben. Damit erhält man an der Meßelektrode 66 eine Reduktion des im Meßdialysat enthaltenen Sauerstoffs zunächst zu H₂O₂ und anschließend zu H₂O nach folgenden Reaktionsgleichungen:

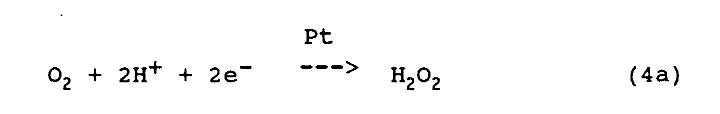

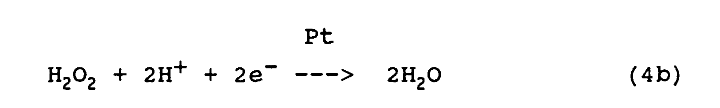

Zur Messung des Sauerstoffpartialdrucks im Meßdialysat wird also der Differenzgrenzstrom bei konstanter Spannung zwischen Meßkathode 66 und Bezugsanode 64 als Maß für die in der Zeiteinheit die Kathode erreichenden O₂-Moleküle genutzt.

Zusammenfassend ist folgendes festzustellen: Die Erfindung bezieht sich auf eine Anordnung zur kontinuierlichen Überwachung der Konzentration von Metaboliten, wie Glukose oder Milchsäure, in Biogeweben, wobei einer vorzugsweise im Subkutangewebe 10 implantierten Mikrodialysesonde 18 ein Perfusatstrom zugeführt und unter Anreicherung mit dem in der Gewebeflüssigkeit enthaltenen Metaboliten als Dialysatstrom entnommen wird. Zusätzlich wird dem Dialysatstrom an einer Mischstelle 20 ein kontinuierlicher Enzymlösungsstrom zugemischt. Mit dem so gebildeten Meßdialysatstrom wird ein elektrochemischer Meßsensor 26 kontinuierlich beaufschlagt und dabei die Konzentration des Metaboliten im Meßdialysatstrom unter selektiver Einwirkung des Enzyms 84 amperometrisch bestimmt.

## Patentansprüche

1. Anordnung zur kontinuierlichen Überwachung der Konzentration eines Metaboliten, wie Glukose oder Milchsäure, in Biogeweben, mit einer im Subkutangewebe (10) implantierbaren, über eine Perfusatleitung (12) mit einem Perfusatstrom beaufschlagbaren Mikrodialysesonde (18), einer den mit dem Metaboliten angereicherten Perfusatstrom als Dialysatstrom von der Mikrodialysesonde (18) abführenden Dialysatleitung (14), und einer vorzugsweise extrakorporal angeordneten, mit der Dialysatleitung (14) verbundenen Meßanordnung (28), welche einen zur Bestimmung der Konzentration des unter der Einwirkung des Enzyms (84) oxidierbaren Metaboliten ausgebildeten Meßsensor (26) aufweist, **dadurch gekennzeichnet, daß** die Dialysatleitung (14) an einer Mischstelle (20) aus einem Enzymlösung (38) enthaltenden Enzymlösungsreservoir (24) in Abhängigkeit vom Durchsatz der Mikrodialysesonde (18) mit einem Enzymlösungsstrom beaufschlagt ist, wobei das Verhältnis zwischen Perfusatstrom und Enzymlösungsstrom 1:10 bis 10:1, vorzugsweise 1:1 beträgt.

2. Anordnung nach Anspruch 1, **gekennzeichnet durch** einen mit dem Enzymlösungsreservoir (24) verbundenen, stromauf des Meßsensors (26) an der Mischstelle (20) gemeinsam mit der Dialysatleitung (14) in eine zu dem Meßsensor (26) führende Meßdialysatleitung (15) mündenden Zuführkanal (22).

3. Anordnung nach Anspruch 1 oder 2, **gekennzeichnet durch** eine in der Perfusatleitung (12) angeordnete, eingangsseitig mit einem Perfusatreservoir (32) und ausgangsseitig mit der Mikrodialysesonde (18) kommunizierende, einen konstanten Perfusatstrom erzeugende erste Fördereinheit (34).

4. Anordnung nach einem der Ansprüche 1 bis 3, **gekennzeichnet durch** eine stromab der Mischstelle (20) vor oder nach dem Meßsensor (26) angeordnete, einen aus dem Dialysatstrom und der an der Mischstelle (20) dem Dialysatstrom zuströmenden Enzymlösung (38) gebildeten, konstanten Meßdialysatstrom fördernde zweite Fördereinheit (36).

5. Anordnung nach Anspruch 4, **dadurch gekennzeichnet, daß** die Förderleistung der zweiten Fördereinheit (36) größer, vorzugsweise doppelt so groß wie die Förderleistung der ersten Fördereinheit (34) ist.

6. Anordnung nach Anspruch 4 oder 5, **dadurch gekennzeichnet, daß** die erste und zweite Fördereinheit (34,36) jeweils als Dosierpumpe, vorzugsweise als Rollendosierpumpe oder Kolbenpumpe ausgebildet sind.

7. Anordnung nach einem der Ansprüche 4 bis 6, **dadurch gekennzeichnet, daß** die erste und zweite Fördereinheit (34,36) als in der Perfusat- und Meßdialysatleitung (12,15) angeordnete, durch einen gemeinsamen Rollkolben zusammendrückbare Pumpschläuche einer einzelnen Rollendosierpumpe (48) ausgebildet sind.

8. Anordnung nach einem der Ansprüche 2 bis 7, **dadurch gekennzeichnet, daß** der in eine Durchflußkammer (16) der Meßdialysatleitung (15) eingreifende, für amperometrische Messungen ausgebildete Meßsensor (26) eine vorzugsweise aus Platin oder Gold bestehende Meßelektrode (66) und eine der Meßelektrode (66) benachbarte, vorzugsweise aus Silber oder Edelstahl bestehende Vergleichselektrode (64) aufweist.

9. Anordnung nach Anspruch 8, **dadurch gekennzeichnet, daß** der Meßsensor (26) eine die Elektroden (64,66) vom Meßdialysatstrom trennende, zweckmäßig auswechselbare semipermeable Schutzmembran (74) aufweist.

10. Anordnung nach Anspruch 8 oder 9, **gekennzeichnet durch** eine die Temperatur in der Durchflußkammer (16) auf einen vorzugsweise 29°C betragenden Vorgabewert einregelnde Temperaturregeleinrichtung.

11. Anordnung nach einem der Ansprüche 1 bis 10, **dadurch gekennzeichnet, daß** in der Dialysatleitung (14) ein bei Rückströmung von Meßdialysat in Richtung der Mikrodialysesonde (18) selbsttätig sperrendes Rückschlagventil angeordnet ist.

12. Anordnung nach einem der Ansprüche 1 bis 11, **dadurch gekennzeichnet, daß** die zweckmäßig tragbare Meßanordnung (28) eine das Meßsignal des Meßsensors (26) aufnehmende und daraus mit der Konzentration des Metaboliten im Gewebe (10) korrelierte digitale Einzeldaten generierende, vorzugsweise mikroprozessorgesteuerte Auswerteelektronik (82) aufweist.

13. Anordnung nach Anspruch 12, **dadurch gekennzeichnet, daß** die Meßanordnung (28) ein die Einzeldaten in kontinuierlichen Zeitabständen abspeicherndes Speichermedium (80) und ein die Einzeldaten anzeigendes, vorzugsweise als LCD-Anzeige ausgebildetes Anzeigemittel (78) umfaßt.

14. Anordnung zur kontinuierlichen Überwachung der Konzentration eines Metaboliten, wie Glukose oder Milchsäure, in Biogeweben, mit einer im Subkutangewebe (10) implantierbaren, über eine Perfusatleitung (12) mit einem Perfusatstrom beaufschlagbaren Mikrodialysesonde (18), einer den mit dem Metaboliten angereicherten Perfusatstrom als Dialysatstrom von der Mikrodialysesonde (18) abführenden Dialysatleitung (14), und einer vorzugsweise extrakorporal angeordneten, mit der Dialysatleitung (14) verbundenen Meßanordnung (28), welche einen zur Bestimmung der Konzentration des unter der Einwirkung des Enzyms (84) oxidierbaren Metaboliten ausgebildeten Meßsensor (26) aufweist, wobei die Dialysatleitung (14) oder/und Perfusatleitung (12) an einer Mischstelle (20) aus einem Enzymlösungsreservoir (24) mit einer Enzymlösung (38) beaufschlagbar ist, **gekennzeichnet durch** eine stromab der Mischstelle (20) vor oder nach dem Meßsensor (26) angeordnete, einen aus dem Dialysatstrom und der an der Mischstelle (20) dem Dialysatstrom zuströmenden Enzymlösung (38) gebildeten, konstanten Meßdialysatstrom fördernde zweite Fördereinheit (36).

## Claims

1. An apparatus for continuously monitoring the concentration of a metabolite, such as glucose or lactic acid, in biological tissue, comprising a microdialysis probe (18) implantable in the subcutaneous tissue (10) and subjectable to a perfusion fluid by way of a perfusion fluid tube (12), a dialysate tube (14) for removing the perfusion fluid enriched with the metabolite as a dialysate flow from the microdialysis probe (18), and a measuring device (28) which is preferably positioned ex vivo, connected to the dialysate tube (14), and has a sensor (26) for determining the concentration of the metabolite oxidizable in the presence of the enzyme (84), **characterized in that** the dialysate tube (14) at a junction (20) is subjected dependent on the through put of the microdialysis probe (18) to an enzyme solution flow from an enzyme solution reservoir (24) containing enzyme solution (38), wherein the ratio of the perfusion fluid flow to the enzyme solution flow lies in the range of 1:10 to 10:1, and is specifically 1:1.

2. The apparatus of claim 1, **characterized by** a feed channel (22) connected to the enzyme solution reservoir (24) and merging with a measuring dialysate tube (15) leading to the sensor (26) together with the dialysate tube (14) at the junction (20) upstream from the sensor (26).

3. The apparatus of claim 1 or 2, **characterized by** first transport means (34), which is positioned in the perfusion fluid tube (12) and communicates with the perfusion fluid reservoir (32) on the intake side and the microdialysis probe (18) on the outlet side producing a constant perfusion fluid flow.

4. The apparatus of one of the claims 1 through 3, **characterized by** second transport means (36) which is positioned downstream from the junction (20) before or after the sensor (26), and which transports a constant measuring dialysate flow formed by the dialysate flow and the enzyme solution (38) joining the dialysate flow at the junction (20).

5. The apparatus of claim 4, **characterized in that** the capacity of the second transport means (36) is larger than the capacity of the first transport means (34), preferably twice as large.

6. The apparatus of claim 4 or 5, **characterized in that** the first and second transport means (34, 36) are designed to be metering pumps, specifically rolling pumps or piston pumps.

7. The apparatus of one of the claims 4 through 6, **characterized in that** the first and second transport means (34, 36) are positioned in the perfusion fluid and measuring dialysate tubes (12, 15), and are designed to be tubes of a single rolling pump (48) compressible by a mutual roller.

8. The apparatus of one of the claims 2 through 7, **characterized in that** the sensor (26), which extends into a flow chamber (16) of the measuring dialysate tube (15) and is designed for amperometric measurements, comprises a measuring electrode (66) consisting of specifically platinum or gold and a comparative electrode (64) consisting of specifically silver or stainless steel and positioned adjacent to the measuring electrode (66).

9. The apparatus of claim 8, **characterized in that** the sensor (26) comprises a semipermeable protection membrane (74) separating the electrodes (64, 66) from the measuring dialysate flow and being advantageously exchangeable.

10. The apparatus of claim 8 or 9, **characterized by** temperature control means regulating the temperature in the flow chamber (16) to a preset value of preferably 29°C .

11. The apparatus of one of the claims 1 through 10, **characterized in that** a check valve which self-locks upon backflow of measuring dialysate in the direction of the microdialysis probe (18) is positioned in the dialysate tube (14).

12. The apparatus of one of the claims 1 through 11, **characterized in that** the advantageously portable measuring device (28) comprises microprocessor-controlled analyzing electronics (82) which record the signal of the sensor (26) and generate therefrom digital data correlated with the concentration of the metabolite in the tissue (10).

13. The apparatus of claim 12, wherein the measuring device (28) comprises storage means (80) storing the data at continuous intervals and display means (78), specifically designed as an LCD-display, displaying the data.

14. An apparatus for continuously monitoring the concentration of a metabolite, such as glucose or lactic acid, in biological tissue, comprising a microdialysis probe (18) implantable in the subcutaneous tissue (10) and subjectable to a perfusion fluid by way of a perfusion fluid tube (12), a dialysate tube (14) for removing the perfusion fluid enriched with the metabolite as a dialsysate flow from the microdialysis probe (18), and a measuring device (28) which is preferably positioned ex vivo, connected to the dialysate tube (14), and has a sensor (26) for determining the concentration of the metabolite oxidizable in the presence of the enzyme (84), whereby the dialysate tube (14) or/and the perfusion fluid tube (12) at a junction (20) is subjectable to an enzyme solution (38) from an enzyme solution reservoir (24), **characterized by** second transport means (36) which is positioned downstream from the junction (20) before or after the sensor (26) and which transports a constant measuring dialysate flow formed by the dialysate flow and the enzyme solution (38) joining the dialysate flow at the junction.

## Revendications

1. Dispositif de surveillance continue de la concentration d'un métabolite, comme le glucose ou l'acide lactique, dans des tissus biologiques, présentant une sonde de micro-dialyse (18), pouvant être implantée dans le tissu sous-cutané (10) et chargée avec un flux de perfusat par le biais d'une conduite de perfusat (12), une conduite de dialysat (14) transportant le flux de perfusat enrichi du métabolite depuis la sonde de micro-dialyse (18) en tant que flux de dialysat, et un dispositif de mesure (28), de préférence disposé extra-corporellement et relié à la conduite de dialyse (14), lequel présente un capteur de mesure (26) conçu pour déterminer la concentration du métabolite pouvant être oxydé sous l'action de l'enzyme (84), **caractérisé en ce que** la conduite de dialysat (14) est chargée par un flux de solution enzymatique au niveau d'un point de mélange (20) depuis un réservoir de solution enzymatique (24) contenant une solution enzymatique (38), en fonction du débit de la sonde de micro-dialyse, le rapport entre flux de perfusat et flux de solution enzymatique étant compris entre 1:10 et 10:1, et étant de préférence égal à 1:1.

2. Dispositif selon la revendication 1, **caractérisé par** un canal d'alimentation (22) relié au réservoir de solution enzymatique (24), et débouchant conjointement à la conduite de dialysat (14) en amont du capteur de mesure (26) au niveau du point de mélange (20) dans une conduite de dialysat de mesure (15) menant au capteur de mesure (26).

3. Dispositif selon la revendication 1 ou 2, **caractérisé par** une première unité transporteuse (34) disposée dans la conduite de perfusat (12), communiquant côté entrée avec un réservoir de perfusat (32) et côté sortie avec la sonde de micro-dialyse (18), et générant un flux de perfusat constant.

4. Dispositif selon l'une des revendications 1 à 3, **caractérisé par** une seconde unité transporteuse (36) disposée en aval du point de mélange (20) avant ou après le capteur de mesure (26), et transportant un flux de dialysat de mesure constant formé du flux de dialysat et de la solution enzymatique (38) s'écoulant en plus du flux de dialysat au niveau du point de mélange (20).

5. Dispositif selon la revendication 4, **caractérisé en ce que** la puissance de déplacement de la seconde unité transporteuse (36) est plus grande, de préférence deux fois plus grande, que la puissance de déplacement de la première unité transporteuse (34).

6. Dispositif selon la revendication 4 ou 5, **caractérisé en ce que** la première et la seconde unités transporteuses (34, 36) sont respectivement conçues en tant que pompe de dosage, de préférence en tant que pompe de dosage à rouleaux ou en tant que pompe à piston.

7. Dispositif selon l'une des revendications 4 à 6, **caractérisé en ce que** la première et la seconde unités transporteuses (34, 36) sont conçues en tant que tubulures de pompe d'une pompe de dosage à rouleaux unique (48), disposées dans la conduite de perfusat et de dialysat de mesure (12, 15) et pouvant être comprimées par un piston à rouleaux conjoint.

8. Dispositif selon l'une des revendications 2 à 7, **caractérisé en ce que** le capteur de mesure (26), pénétrant dans une chambre de circulation (16) de la conduite du dialysat de mesure (15), et conçu pour des mesures ampérométriques, présente une électrode de mesure (66) de préférence composée de platine ou d'or, et une électrode de référence (64) voisine de l'électrode de mesure (66) et de préférence composée d'argent ou d'acier spécial.

9. Dispositif selon la revendication 8, **caractérisé en ce que** le capteur de mesure (26) présente une membrane protectrice (74) semi-perméable pouvant être remplacée de façon appropriée et séparant les électrodes (64, 66) du flux du dialysat de mesure,.

10. Dispositif selon la revendication 8 ou 9, **caractérisé par** une unité de régulation de la température, réglant la température dans la chambre de circulation (16) à une valeur prédéfinie de préférence égale à 29 °C.

11. Dispositif selon l'une des revendications 1 à 10, **caractérisé en ce qu'**une soupape de retenue, auto-bloquante en cas de reflux du dialysat de mesure dans la direction de la sonde de micro-dialyse (18), est disposée dans la conduite de dialysat (14).

12. Dispositif selon l'une des revendications 1 à 11, **caractérisé en ce que** le dispositif de mesure (28), de façon appropriée portable, comporte un dispositif électronique d'analyse (82), de préférence commandé par micro-processeur, qui enregistre le signal de mesure du capteur de mesure (26) et génère à partir de celui-ci des données numériques corrélées à la concentration du métabolite dans le tissu (10).

13. Dispositif selon la revendication 12, **caractérisé en ce que** le dispositif de mesure (28) comprend un moyen de mémorisation (80) stockant les données selon des intervalles continus, et un moyen d'affichage (78) qui indique les différentes données, conçu de préférence en tant qu'écran LCD.

14. Dispositif de surveillance continue de la concentration d'un métabolite, comme le glucose ou l'acide lactique, dans des tissus biologiques, présentant une sonde de micro-dialyse (18), pouvant être implantée dans le tissu sous-cutané (10) et chargée avec un flux de perfusat par le biais d'une conduite de perfusat (12), une conduite de dialysat (14) transportant depuis la sonde de micro-dialyse (18) le flux de perfusat enrichi du métabolite en tant que flux de dialysat, et un dispositif de mesure (28), de préférence disposé extra-corporellement et relié à la conduite de dialyse (14), lequel présente un capteur de mesure (26) conçu pour déterminer la concentration du métabolite pouvant être oxydé sous l'action de l'enzyme (84), la conduite de dialysat (14) et/ou la conduite de perfusat (12) pouvant être alimentées avec une solution enzymatique (38) issue d'un réservoir de solution enzymatique (24) au niveau d'un point de mélange (20), **caractérisé par** une seconde unité transporteuse (36), disposée en aval du point de mélange (20) avant ou après le capteur de mesure (26), et transportant un flux de dialysat de mesure constant formé du flux de dialysat et de la solution enzymatique (38) ajoutée au flux de dialysat au niveau du point de mélange (20).
